# EUROPEAN PATENT SPECIFICATION

(11) **EP 0 606 391 B1**
(45) Date of publication and mention of the grant of the patent: **05.08.1998**
(21) Application number: 92922039.0
(22) Date of filing: 30.09.1992
(51) Int. Cl.: A61K 39/395

(54) **INHIBITION OF VASCULAR NARROWING USING ANTI-PADGEM ANTIBODIES**
Hemmung von Gefässverengung unter Verwendung von anti-PADGEM Antikörpern.
INHIBITION DU RETRECISSEMENT VASCULAIRE PAR L'UTILISATION D'ANTICORPS ANTI-PADGEM

(30) Priority: 30.09.1991 US 768834
(43) Date of publication of application: 20.07.1994
(73) Proprietor: BIOGEN, INC., Cambridge Massachusetts 02142 (US); NEW ENGLAND MEDICAL CENTER, Boston, MA 02111 (US)
(72) Inventor: PALABRICA, Theresa, M., Hingham, MA 02043 (US); FURIE, Bruce, E., Wellesley, MA 02043 (US); FURIE, Barbara, C., Wellesley, MA 02043 (US); LOBB, Roy, R., Westwood, MA 02090 (US); BENJAMIN, Christopher, D., Beverly, MA 01915 (US)
(74) Representative: Hallybone, Huw George
(86) International application number: US9208163
(87) International publication number: WO9306863

(56) References cited:
- WO-A-91/01380
- WO-A-91/06632
- WO-A-91/07993
- CURRENT STUDIES IN HEMATOLOGY & BLOOD TRANSFUSION, vol. 58, 16 June 1991, Basel (CH); B. FURIE et al., 32-36/
- PROCEEDINGS OF THE NATL. ACADEMY OF SCIENCES USA, vol. 86, no. 3, 01 February 1989, Washington, DC (US); T. PALABRICA et al., pp. 1036-1040/
- CIRCULATION, vol. 83, no. 1, January 1991, New York, NY (US); D. MILLER et al., pp. 224-236/
- PROCEEDINGS OF THE NATL. ACADEMY OF SCIENCES USA, vol. 84, no. 21, November 1987, Washington, DC (US); M. RUNGE et al., pp. 7659-7662/
- Circulation, Vol.84 (1991) p.232-243

## Description

### FIELD OF THE INVENTION

The present invention relates to the inhibition of vascular narrowing at an intravascular site treated by angioplasty, endarterectomy or atherectomy, in natural and synthetic vascular grafts, in arteriovenous fistulas, at intravascular sites following deployment of vascular stents or cardiovascular devices, or at an intravascular site of platelet deposition. More particularly, this invention relates to the use of antibodies recognizing Platelet Activation Dependent Granule-External Membrane protein ("PADGEM") and antibody homologs thereof to inhibit these vascular narrowings.

### BACKGROUND OF THE INVENTION

Several interventional therapeutic procedures are available to an attending physician for use in treating occlusive artery disease. These procedures include balloon angioplasty, endarterectomy, atherectomy, bypass surgery, deployment of vascular stents and insertion of cardiovascular devices.

Angioplasty procedures typically involve dilation of a blood vessel by means of a balloon catheter inserted through the lumen of the affected vessel to the site of vessel narrowing. When the balloon catheter reaches the narrowed segment of the vessel, the balloon is inflated to flatten occlusive material against the vessel wall. The object of angioplasty is to widen the lumen of the affected vessel to increase blood flow. Percutaneous transluminal coronary angioplasty ("PTCA") is a type of angioplasty procedure commonly used in treating occluded coronary arteries. Angioplasty is also used to increase blood flow in occluded peripheral blood vessels.

Like angioplasty, endarterectomy and atherectomy procedures are used to treat occlusive artery disease. Endarterectomy is a surgical procedure involving the excision of the thickened, atheromatous tunica intima of an artery. Carotid endarterectomy is an example of this procedure in which occlusive material is removed from the carotid artery to increase blood flow to the brain.

In atherectomy, a cutting device attached to a catheter is positioned at a site of atherosclerotic plaque deposition. When in position, the cutting device is activated to disrupt and collect the plaque. This procedure may be used to treat occlusions in peripheral and coronary vessels.

Bypass surgery involves the insertion of a vascular graft prosthesis to bypass occluded, narrowed or injured segments of arteries or vasculature. Grafting may be accomplished using natural vascular tissue or synthetic materials, such as polytetrafluroethylene ("PTFE"). The successful graft restores blood flow to deprived tissues. Coronary artery bypass graft surgery ("CABG") is used to restore blood flow to deprived coronary tissues. Bypass graft surgery may also be used to restore blood to peripheral tissues.

Balloon angioplasty, endarterectomy, atherectomy and bypass surgery are costly and invasive procedures that present significant risks to the patient. Complications associated with these procedures have been well documented and often involve narrowing of the treated vessel.

Post-angioplasty, post-endarterectomy and post-atherectomy vascular narrowing may result from acute reclosure of the vessel shortly after the procedure, or from gradual, progressive restenosis of the vessel. Significant restenosis occurs, for example, in one third of all patients undergoing coronary angioplasty and carotid endarterectomy. Repeated procedures or bypass surgery are often necessary to treat these vascular narrowings.

Total closure and progressive stenosis of the vascular graft prosthesis are also known to occur following bypass surgery. These complications may likewise require additional treatment with angioplasty or repeated bypass surgery.

Other interventional therapeutic procedures for symptomatic occlusive artery disease, such as deployment of vascular stents and insertion of cardiovascular devices, are also complicated by vascular narrowing. Vascular stents are devices that are used to hold blood vessels open by physical means. Stents may be positioned in peripheral and coronary arteries using specially designed flexible delivery catheters. Stent placement is often performed during an angioplasty procedure. Severe cardiovascular disease may require treatment with cardiovascular devices, including prosthetic heart valves and artificial hearts. Thrombotic occlusion and stenosis of blood vessels have been observed following deployment of vascular stents and cardiovascular devices, often requiring repeated surgery and replacement of the device.

Temporary or permanent arteriovenous fistulas, which are implant sites used to accomplish vascular access for hemodialysis, are also often complicated by implant narrowing. This narrowing may result from early thrombotic occlusion or from progressive stenosis.

Deposition of platelets at the site of vascular narrowing in cases of reclosure and restenosis of vessels and grafts following interventional therapeutic procedures has been observed. It is not clear what effect platelet deposition may have on reclosure and restenosis. Deposition of platelets may be associated with the formation of thrombi and may be a physiological response to vascular tissue injury. The possible activation of the deposited platelets and their production of PADGEM may also affect leukocyte recruitment.

### SUMMARY OF THE INVENTION

The present invention provides the use of an anti-PADGEM antibody, recombinant antibody, chimeric antibody or humanized antibody, or Fab fragment, Fab' fragment, F(ab')₂ fragment, F(v) fragment, heavy chain monomer, heavy chain dimer, heavy chain trimer, light chain monomer, light chain dimer, light chain trimer or dimer between one light chain and one heavy chain thereof that binds to PADGEM or a peptide that mimics the activity of anti-PADGEM antibodies in the manufacture of a medicament for inhibiting vascular narrowing at intravascular sites treated by angioplasty, endarterectomy or atherectomy, in natural and synthetic vascular grafts, at intravascular sites following deployment of vascular stents or cardiovascular devices, in arteriovenous fistulas, or at an intravascular site of platelet deposition.

### BRIEF DESCRIPTION OF THE FIGURES

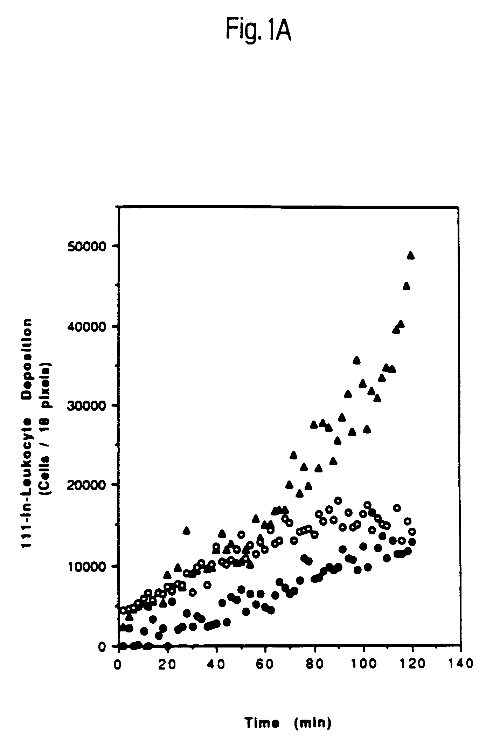

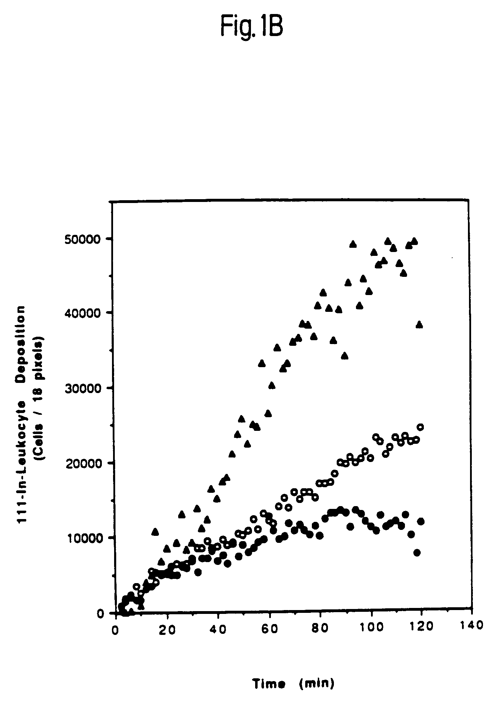

Figures 1A and 1B depict serial anterior images of the external shunt implanted in two baboon subjects (baboons A and B). The images were attained using an Ohio Nuclear Series 100 gamma camera. These images demonstrate that neutrophil and monocyte accumulation in the shunt were depressed following the infusion of monoclonal antibody GA6 or GA6 F(ab 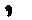 )₂ fragments. ▲ represents leukocyte deposition when no antibody was infused; ○ represents leukocyte deposition following GA6 antibody infusion; and ● represents leukocyte deposition following GA6 F(ab 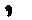 )₂ fragment infusion.

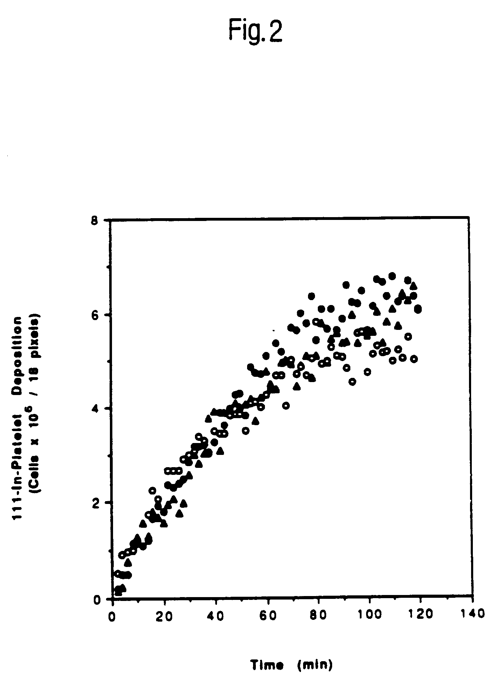

Figure 2 depicts serial anterior images of the shunt and demonstrates that the rate and quantity of platelet deposition were substantially identical in the presence and absence of infused GA6 antibody or GA6 antibody fragments. ▲ represents platelet deposition where no antibody was infused; ○ represents platelet deposition following GA6 antibody infusion; and ● represents platelet deposition following GA6 F(ab 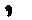 )₂ fragment infusion.

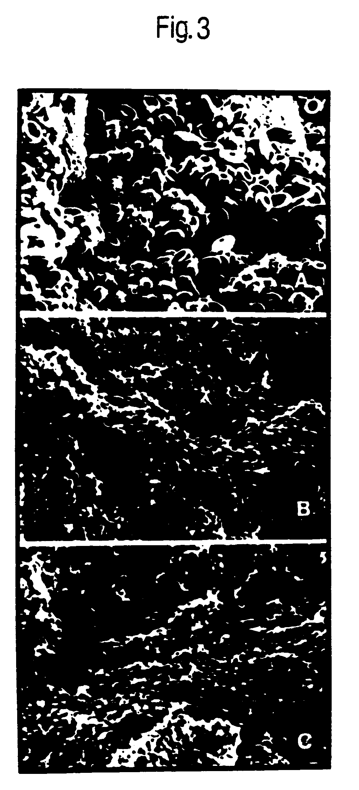

Figure 3 shows representative scanning electron micrographs of the surface of the lumen of the Dacron graft removed from the arteriovenous shunt. Shunts obtained from a baboon not infused with antiPADGEM antibodies or antibody fragments had considerable amounts of fibrin clot, platelets and leukocytes. Shunts obtained from a baboon infused with anti-PADGEM antibodies or antibody fragments had platelets, but the amount of fibrin clot and leukocytes were very significantly reduced. Figure 3A shows the surface of the lumen when no anti-PADGEM GA6 antibodies were infused. The arrow indicates a bound leukocyte. Figure 3B shows the surface of the lumen when GA6 antibodies were infused. The arrow indicates a bound platelet. Figure 3C shows the surface of the lumen when F(ab 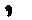 )₂ fragments of GA6 were infused. The arrow indicates a bound erythrocyte. The micrographs were derived from Dacron grafts removed from baboon B after the completion of each experiment

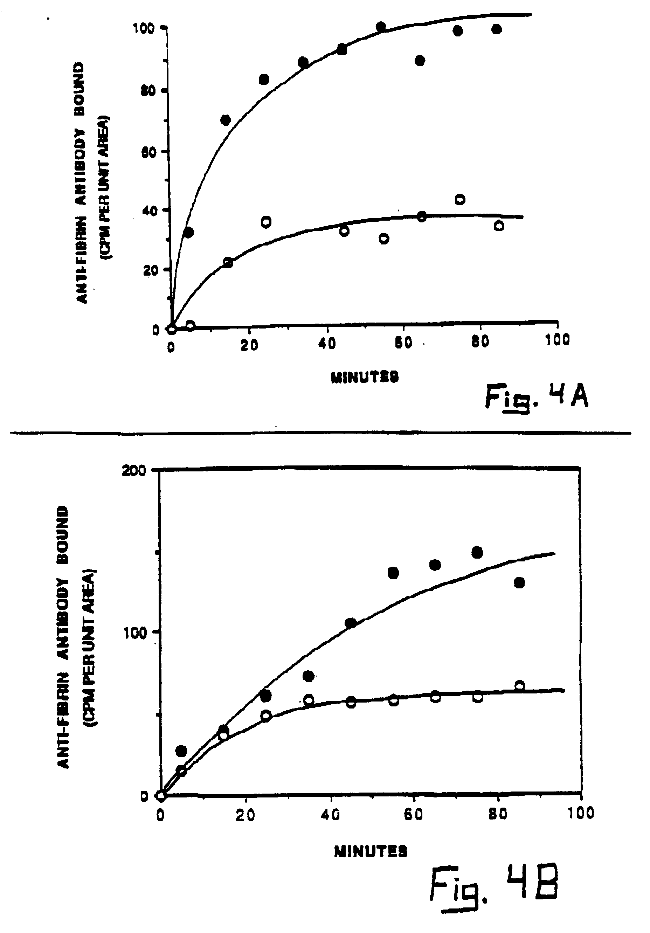

Figures 4A and 4B. Fibrin deposition on the Dacron craft and its inhibition by the anti-P-selectin GA6 antibody. The Fab' fragment of T2G1s (a fibrin-specific antibody), labeled with technetium-99m, was injected via the venous connector of the shunt just prior to the initiation of flow while, where indicated, GA6 Fab'₂ was injected simultaneously via the arterial connector. 4A Baboon A, with (○) or without (●) GA6 Fab'₂ antibodies (2 mg/kg). B. Baboon B, with (○) or without (●) GA6 Fab'₂ antibodies (5 mg/kg).

### DETAILED DESCRIPTION

PADGEM, a 140,000 Dalton glycoprotein, is a component of the membrane of alpha granules and Weibel-Palade bodies of platelets and endothelial cells (Berman et al. 1986 [1], Stenberg et al. 1985 [2], Bonfanti et al. 1989 [3]). PADGEM is expressed on the plasma membrane of activated platelets (Larsen et al. 1989 [4], Hamburger and McEver 1990 [5]). It is thought to mediate cellular adhesion of activated platelets to neutrophils and monocytes [5]. WO 91/01380, WO 91/06632 and "Current Studies in Hematology and Blood Transfusion, No. 58 (June 17, 1991) p.32-36" disclose an inhibition of the interaction between leukocytes and activated platelets by the use of anti-PADGEM antibodies.

The present invention is directed to the use of an anti-PADGEM antibody, recombinant antibody, chimeric antibody or humanized antibody, or Fab fragment, Fab' fragment, F(ab')₂ fragment, F(v) fragment, heavy chain monomer, heavy chain dimer, heavy chain trimer, light chain monomer, light chain dimer, light chain trimer or dimer between one light chain and one heavy chain thereof that binds to PADGEM or a peptide that mimics the activity of anti-PADGEM antibodies in the manufacture of a medicament for inhibiting vascular narrowing at intravascular sites treated by angioplasty, endarterectomy or atherectomy, in natural and synthetic vascular grafts, at intravascular sites following deployment of vascular stents or cardiovascular devices, in arteriovenous fistulas, or at an intravascular site of platelet deposition.

As used herein, an "antibody homolog" that binds to PADGEM is a protein or polypeptide comprising one or more polypeptides selected from immunoglobulin light chains, immunoglobulin heavy chains and antigen-binding fragments thereof, that are capable of binding to PADGEM. The component polypeptides of an antibody homolog composed of more than one polypeptide may optionally be disulfide-bound or otherwise covalently crosslinked. Accordingly, antibody homologs that bind to PADGEM include intact immunoglobulins of types IgA, IgG, IgE, IgD, IgM (as well as subtypes thereof), wherein the light chains of the immunoglobulin may be of types kappa or lambda. These antibody homologs also include portions of intact immunoglobulins that retain PADGEM-binding specificity, for example, Fab fragments, Fab' fragments, F(ab')₂ fragments, F(v) fragments, heavy chain monomers, dimers or trimers, light chain monomers, dimers or trimers, dimers consisting of one heavy and one light chain, and the like. Such antibody fragments may be produced, for example, by chemical methods, e.g., by cleaving an intact antibody with a protezse, such as pepsin or papain, and optionally treating the cleaved product with a reducing agent. Alternatively, useful fragments may be produced by using host cells transformed with truncated heavy and/or light chain genes. Heavy and light chain monomers nay be produced by treating an intact antibody with a reducing agent, such as dithiothreitol, followed by purification to separate the chains. Heavy and light chain monomers may also be produced by host cells transformed with DNA encoding either the desired heavy chain or light chain, but not both (see, e.g., Ward et al., 1989 [6]; L. Sastry et al., 1989 [7]).

Antibody homologs useful in the present invention also include peptides that mimic the activity of anti-PADGEM antibodies by binding PADGEM. Such mimetic peptides may be produced by synthesizing a plurality of peptides, semi-peptidic compounds or non-peptidic compounds and then screening those compounds for their ability to bind PADGEM and to inhibit leukocyte binding to PADGEM. (See e.g. Scott and Smith, 1990 [8]; Devlin et al., 1990 [9]).

The antibody homologs useful in the present invention also will include humanized, chimeric and recombinant antibodies that bind to PADGEM. (See, e.g., Boss, United States patent 4,816,397 [10]). For example, recombinant antibodies may be produced by cloning cDNA or genomic DNA encoding the immunoglobulin light and heavy, chains of the desired antibody from a hybridoma cell that produces a desired antibody. The cDNA or genomic DNA encoding those polypeptides is then inserted into expression vectors so that both genes are operatively linked to transcriptional and translational expression control sequences. The expression vector and expression control sequences are chosen to be compatible with the expression host cell used. Typically, both genes are inserted into the same expression vector.

Prokaryotic or eukaryotic host cells may be used. Expression in eukaryotic host cells is preferred because such cells are more likely than prokaryotic cells to assemble and secrete a properly folded and immunologically active antibody. However, any antibody produced that is inactive due to improper folding may be renaturable according to well-known methods (see Kim and Baldwin, 1982 [11]). It is possible to use the host cells to produce only portions of intact antibodies, such as light chain dimers or heavy chain dimers, which also are antibody homologs useful in the present invention.

It will be understood that variations on the above procedures are also contemplated in the present invention. For example, it may be desired to transform a host cell with DNA encoding either the light chain or the heavy chain (but not both) of an antibody homolog. Recombinant DNA technology may also be used to remove some or all of the DNA encoding either or both of the light and heavy chains that is not necessary for PADGEM binding. The molecules expressed from such truncated DNA molecules are antibody homologs according to this invention. In addition, bifunctional antibodies may be produced in which one heavy and one light chain are antibody homologs of this invention and the other heavy and light chain are specific for an antigen other than PADGEM, or another epitope of PADGEM.

Chimeric recombinant antibody homologs that bind to PADGEM may also be used in this invention. As used herein, a "chimeric recombinant antibody" homolog is an antibody homolog derived initially from a nonhuman mammalian antibody, wherein recombinant DNA technology has been used to replace all or part of the hinge and constant regions of the light chain, the heavy chain or both, with corresponding regions from a human immunoglobulin light chain or heavy chain.

Chimeric recombinant antibodies are produced by transforming a host cell with a suitable expression vector comprising DNA encoding the desired immunoglobulin light and heavy chains in which all or some of the DNA encoding the hinge and constant regions of the heavy and/or the light chain have been substituted with DNA from the corresponding region of an immunoglobulin light or heavy chain of a different species. When the original recombinant antibody is nonhuman, substitution of corresponding human sequences is preferred. An exemplary chimeric recombinant antibody has mouse variable regions and human hinge and constant regions. (See generally, Boss et al., U.S. patent 4,816,397 [10]; Cabilly et al., U.S. patent 4,816,567 [12]; and Morrison et al., 1984 [13]).

Anti-PADGEM antibodies useful in this invention are preferably monoclonal antibodies. They may be produced using conventional materials and methods. Anti-PADGEM monoclonal antibodies have been described previously (e.g., Larsen et al., 1989 [4]; Furie, U.S. Patent 4,783,330 [14]). The anti-PADGEM antibodies described in United States patent 4,783,330 were deposited under ATCC accession No. HB 8670. For the demonstrations described in the example below, an anti-PADGEM monoclonal antibody designated GA6 was used. Other anti-PADGEM antibodies also will be useful in the present invention. These include monoclonal antibodies GE12 and AC11 which, like GA6, bind with PADGEM and inhibit leukocyte binding to PADGEM. Cell lines producing these antibodies were deposited with American Type Culture Collection (ATCC), Rockville, MD. The ATCC designation numbers for cell lines producing respectively GA6, GE12 and AC11 antibodies are HB10943, HB10942 and HB10941. Monoclonal antibodies GA6, GE12 and AC11 may be prepared in the manner described below.

The technology for producing monoclonal antibodies is well known (see generally Lerner, 1981 [15]; Gefter et al., 1977 [16]). Briefly, an immortal cell line (typically myeloma cells) is fused to lymphocytes (typically splenocytes) from a mammal immunized with a preparation comprising PADGEM. The culture supernatants of the resulting hybridoma cells are screened for those antibodies having the desired properties, e.g., binding to PADGEM.

Any of the many well known protocols used for fusing lymphocytes and immortal cell lines are useful for generating such monoclonal antibodies (see, e.g., Galfre et al., (1977) [17]; Gefter et al., 1977 [16]). Many variations of such methods are known to those skilled in the art and will also be useful.

Typically (and preferably) the immortal cell line (e.g., a myeloma cell line) is derived from the same mammalian species as the lymphocytes. Useful mammals include mice and rats. More preferably, both the immortal cell line and the lymphocytes are derived from an inbred mouse of strain BALB/c (Jackson Labs, Bar Harbour, ME). Preferred immortal cell lines are mouse myeloma cell lines that are sensitive to culture medium containing hypoxanthine, aminopterin and thymidine ("HAT medium"). The most preferred mouse myeloma cell line is P3X63-AG8.653 (ATCC, Rockville, MD, catalog no. CRL 1580).

In that preferred embodiment, HAT-sensitive mouse myeloma cells are fused to mouse splenocytes using polyethylene glycol ("PEG"), preferably PEG-3350. Immunization of the mammal in the production of these monoclonal antibodies may be accomplished using standard procedures. PADGEM fragments, intact PADGEM molecules and whole activated platelets may be used for immunization. The unit dose and immunization regimen depend on the species of animal immunized, its immune status, the body weight of the mammal, and the PADGEM concentration in the PADGEM preparation administered. We prefer, for example, to immunize Balb/c mice monthly for three months with 12 µg of PADGEM. For the initial immunization, PADGEM is typically emulsified in complete Freund's adjuvant, and for subsequent immunizations, incomplete Freund's adjuvant is used. Prior to fusion, the mice may be boosted with PADGEM (e.g., 12 µg) in incomplete Freund's adjuvant.

The immunized mammals may be bled between boosts and the serum from each blood sample assayed for the desired monoclonal antibodies using any of the screening assays described below. The lymphocytes used in the production of hybridoma cells typically are isolated from immunized mammals whose sera have already tested positive for the presence of monoclonal antibodies that bind to PADGEM.

Hybridoma cells resulting from the fusion of the immortalized mouse cells are then selected using HAT medium, which kills unfused and unproductively fused myeloma cells (unfused splenocytes die after several days because they are not transformed).

Hybridoma cells producing anti-PADGEM monoclonal antibodies may be detected by screening the hybridoma culture supernatants using various conventional screening assays. Assays to determine whether a particular antibody binds to PADGEM and whether a particular antibody inhibits PADGEM binding to leukocytes, e.g., HL60 cells, are preferred. Appropriate assays include those based upon antibody binding to PADGEM on plates using ELISA, those based on antibody binding to activated platelets using FACS; adhesion assays for antibody inhibition of leukocyte (e.g., HL60) cell binding to PADGEM; and adhesion assays for antibody inhibition of leukocyte cell binding to activated platelets.

Hybridomas testing positive for the desired antibody activity may be cloned by limiting dilution and cultured in a nutrient medium under conditions and for a time sufficient to allow the hybridoma cells to secrete the monoclonal antibodies into the culture medium. Tissue culture techniques and culture media suitable for hybridoma cells are well known (see, e.g., Lerner, [15]). Finally, conditioned hybridoma culture supernatant containing the antibody homologs is collected.

Alternatively, the desired monoclonal antibody may be produced by injecting the hybridoma cells into the peritoneal cavity of an unimmunized mouse. The hybridoma cells proliferate in the peritoneal cavity, secreting the monoclonal antibody, which accumulates as ascites fluid (see Lerner, [15]). The antibody is then harvested by withdrawing the ascites fluid from the peritoneal cavity with a syringe.

Monoclonal antibodies may be purified easily from conditioned media or ascites using techniques well known to the art.

The present invention uses anti-PADGEM antibodies and their antibody homologs to manufacture a medicament which may be used to inhibit vascular narrowing at an intravascular site treated by angioplasty, endarterectomy or atherectomy, in a natural or synthetic vascular graft, in arteriovenous fistulas, at an intravascular site following deployment of vascular stents or cardiovascular devices, or at an intravascular site of platelet deposition. The anti-PADGEM antibodies and antibody homologs useful in this invention are administered in the form of a pharmaceutical composition comprising a therapeutically effective amount of the antibody and, preferably, a pharmaceutically acceptable carrier, such as physiological saline.

An appropriate therapeutic dosage for an anti-PADGEM antibody or antibody homolog of this invention depends on factors such as the half-life of the particular antibody or antibody homolog, the isotope of antibody or antibody homolog, the condition being treated, the age, sensitivity, and tolerance of the patient, and other such factors routinely considered by an attending physician. Appropriate dosages may range from about 0.1 mg/kg of body weight of the patient to about 5 mg/kg, and the dosage may be administered from once per day for a single administration to a constant infusion for several days depending on factors such as those cited above. A dosage of 1 mg/kg for a single administration was, for example, appropriate in the baboon arteriovenous shunt model described in the example below.

The anti-PADGEM antibodies and antibody homologs useful in this invention nay be administered to the patient by any suitable means such as intravenously, intramuscularly, subcutaneously, or intra-arterially. Intravenous administration is preferred.

A cardiologist may advantageously use the medicament manufactured according to the present invention, for example, in circumstances where a patient is at risk for vascular narrowing associated with an interventional therapeutic procedure. Anti-PADGEM monoclonal antibodies or antibody homologs thereof may be therapeutically administered prior to, during and following an angioplasty, endarterectomy or atherectomy procedure, the insertion of a natural or synthetic vascular graft, the placement of arteriovenous fistulas, the deployment of an vascular stent or a cardiovascular device, or an intravascular deposition of platelets. Administration of the anti-PADGEM monoclonal antibodies will effectively inhibit vascular narrowing associated with restenosis, reocclusion and graft closure.

In order that this invention may be better understood, the following example is set forth. This example is for purposes of illustration only, and is not to be construed as limiting the scope of the invention in any manner.

### EXAMPLE

### Inhibition of Vascular Narrowing in an Arteriovenous Shunt Model

Employing an arteriovenous shunt model in baboons, we have demonstrated that the administration of a therapeutically effective amount of anti-PADGEM monoclonal antibodies or antibody fragments is surprisingly effective in inhibiting vascular narrowing at an intravascular site in a vascular graft.

### A. The Monoclonal Antibody

Anti-PADGEM monoclonal antibody GA6 was prepared using Balb/cJ mice. The mice were immunized monthly for 3 months with 12 µg of PADGEM. PADGEM was derived from pooled human platelets using affinity purification. For the initial immunization, PADGEM was emulsified in complete Freund's adjuvant, and for subsequent immunizations, PADGEM was emulsified in incomplete Freund's adjuvant. Four days prior to fusion, the mice were boosted with 12 µg PADGEM in incomplete Freund's adjuvant. Hybridomas were prepared by standard methods using P3X63-Ag8.653 myeloma cells as fusion partners (Lerner, 1981 [15]). Hybridoma culture supernatants were screened for binding to PADGEM-coated plates using ELISA and were screened for inhibition of adhesion of HL60 cell binding to PADGEM-coated plates. Cells putatively positive in these assays were cloned by limiting dilution. Antibodies were isolated from ascites fluid using protein A affinity chromatography.

Antibody GA6 and fragments thereof bind to baboon platelets with high affinity and inhibit binding of HL60 cells to PADGEM in vitro.

### B. The Graft

An ex vivo Dacron graft was affixed to the interior walls of an arteriovenous shunt. Such a graft is thrombogenic and capable of accumulating platelets and fibrin within its lumen. Prior to the surgical introduction of the arteriovenous shunt into the baboons, the Dacron graft was preclotted in whole baboon blood according to the method of Sauvage (Sauvage, 1976 [18]). Nuclear imaging with ¹²³I-labeled polyclonal anti-PADGEM antibodies demonstrated that PADGEM was expressed by the platelets accumulated within the thrombogenic Dacron graft (Palabrica et al., 1989 [19]).

### C. Demonstration of In Vivo Antibody Activity

We analyzed the interaction between the PADGEM expressed on the cell membranes of the bound platelets and the anti-PADGEM antibodies using radiolabeled neutrophils and monocytes from the host baboon.

Leukocytes from baboon whole blood were prepared by density gradient centrifugation. Neutrophils and mononuclear cells were labeled via incubation with ¹¹¹Indium oxine (424 µCi). The labeled cells were washed with plasma and subsequently resuspended in plasma. The ¹¹¹In-labeled leukocytes were infused into anesthetized baboons. One hour later, the preclotted shunt was introduced into the baboons. Serial anterior images of the external shunt attained using an Ohio Nuclear series 100 gamma camera demonstrated that, in the absence of anti-PADGEM antibodies, neutrophils and monocytes accumulated in the shunt. When antibody GA6 or fragments thereof were infused into the baboon at a dosage of 1 mg/kg one hour after the infusion of the ¹¹¹In-labeled leukocytes and one minute after the insertion of the Dacron shunt, serial images demonstrated that neutrophil and monocyte accumulation within the shunt was depressed (see Figures 1A and 1B).

As a control experiment, AC1.2, a non-inhibitory anti-PADGEM monoclonal antibody was compared to GA6 in its effect on leukocyte accumulation on the graft. Infusion of the AC1.2 Fab'₂ fragments (1 mg/kg) into the baboon had no effect on leukocyte accumulation into the Dacron graft inasmuch as the number of bound labeled leukocytes were the same, within experimental error, in the presence or absence of AC1.2 at 90 and 120 minutes following antibody infusion. In contrast to GA6, the AC1.2 Fab'₂ fragments did not inhibit leukocyte accumulation into the Dacron graft, thus demonstrating that only antibodies that inhibit leukocyte/platelet interaction in vitro will inhibit leukocyte accumulation in vivo.

Use of radiolabeled platelets demonstrated that anti-PADGEM antibodies do not affect the accumulation of platelets in the Dacron shunt. Baboon platelets were labeled with ¹¹¹In and were subsequently infused into a baboon. Serial anterior images of the shunt showed that the rate and quantity of platelet deposition were substantially identical in the presence and absencë of infused GA6 antibody or antibody fragments (see Figure 2).

Additional in vivo experiments were performed as above except that a radiolabeled anti-fibrin antibody was monitored for binding to the graft. The Fab' fragment of T2G1s (kindly provided by Dr. Thomas Shabile, Centocor) was labeled with technetium-99m (specific radioactivity 23mCi/mg). The antibody (2 mCi) was injected via the venous connector of the shunt just prior to the initiation of flow while, where indicated. GA6 Fab'₂ was injected simultaneously via the arterial connector during the first minute of flow. Serial two minute anterior images were obtained over the shunt for a 90 minute duration using an Ohio Nuclear series 100 Gamma camera. A medium energy, high resolution, parallel-hole collimator was used with a 20% window. The kinetics of Tc-99m T2G1s antibody uptake by the graft and blood pool was derived from the radionuclide images. The activity was expressed as counts per minute normalized to injected dose and corrected for background activity.

The results are shown in Figure 4. These results demonstrate substantial decrease in fibrin deposition resulting from treatment with the GA6 Fab'₂fragment.

### D. Evaluation of the Shunt

After insertion of the shunt into the baboons, blood flow through the shunt was maintained at 100 ml per minute throughout the course of the experiments. Following the period of blood flow, the shunts were evaluated histopathologically to compare the shunts from animals that were treated with antibody GA6 or GA6 antibody fragments with the shunts from animals that did not receive antibody GA6 or antibody fragments. The shunt was removed, washed, and after fixation in 2% glutaraldehyde, the shunt was sectioned longitudinally and examined by scanning electron microscopy. Shunts obtained from animals not infused with anti-PADGEM antibody or antibody fragments had considerable amounts of fibrin clot, platelets and leukocytes. In contrast, shunts obtained from animals infused with anti-PADGEM antibody, or antibody fragments had platelets, but the amount of fibrin clot and leukocytes associated with the shunt were very significantly reduced (see Figure 3).

The shunt model in baboons demonstrates that, anti-PADGEM antibodies have no effect on the accumulation of platelets within the shunt. However, microscopic evaluation of the shunt surprisingly shows that the anti-PADGEM antibodies or fragments thereof may considerably reduce the inflammatory and thrombotic response within a blood vessel following the deposition of platelets.

The foregoing example, therefore, unexpectedly demonstrates that anti-PADGEM antibodies or antibody fragments may be used to inhibit vascular narrowing at an intravascular site treated by angioplasty, endarterectomy or atherectomy, in natural and synthetic vascular grafts, in arteriovenous fistulas, at intravascular sites following deployment of vascular stents or cardiovascular devices and at intravascular sites of platelet deposition.

A wide variety of anti-PADGEM antibodies and antibody homologs thereof that bind PADGEM, other than the antibody specifically mentioned in the foregoing example, are also useful in the method of the present invention. All such embodiments, in view of the teachings herein, are specifically contemplated and included within the scope of the invention as defined in the following claims.

### CITED PUBLICATIONS

[1] C. L. Berman et al., "A Platelet Alpha Granule Membrane Protein that is Associated with the Plasma Membrane After Activation," J. Clin. Invest., **78**, pp. 130-37 (1986).
[2] P. E. Stenberg et al., "A Platelet Alpha-Granule Membrane Protein (GMP-140) is Expressed on the Plasma Membrane After Activation," J. Cell Biol., **101**, pp. 880-86 (1985).
[3] R. Bonfanti et al., "PADGEM (GMP 140) is a Component of Weibel-Palade Bodies Of Human Endothelial Cells," Blood, **73**, pp. 1109-12 (1989).
[4] E. Larsen, et al., "PADGEM Protein: A Receptor That Mediates the Interaction of Activated Platelets with Neutrophils and Monocytes," Cell, **59**, pp. 305-12 (1989).
[5] S. A. Hamburger and R. P. McEver, "GMP-140 Mediates Adhesion of Stimulated Platelets to Neutrophils," Blood, **75**, pp. 550-554 (1990).
[6] E. S. Ward et al., "Binding Activities of a Repertoire of Single Immunoglobulin Variable Domains Secreted From Escherichia coli," Nature, **341**, pp. 544-46 (1989).
[7] L. Sastry et al., "Cloning of the Immunological Repertoire in Escherichia coli For Generation of Monoclonal Catalytic Antibodies: Construction of a Heavy Chain Variable Region-Specific cDNA Library," Proc. Natl. Acad. Sci. USA, **86**, pp. 5728-32 (1989).
[8] J. K. Scott and G. P. Smith, "Searching for Peptide Ligands with an Epitope Library," Science, **249**, pp. 386-90 (1990).
[9] J. J. Devlin et al., "Random Peptide Libraries: A Source of Specific Protein Binding Molecules," Science, **249**, pp. 404-07 (1990).
[10] U.S. Patent No. 4,816,397, (Boss et al.), "Multichain Polypeptides or Proteins and Processes For Their Production," March 28, 1989.
[11] P. S. Kim and R. L. Baldwin, "Specific Intermediates in the Folding Reactions of Small Proteins and the Mechanism Of Protein Folding," Ann. Rev. Biochem., **51**, pp. 459-89 (1982).
[12] U.S. Patent No. 4,816,567, (Cabilly et al.), "Recombinant Immunoglobin Preparations," March 28, 1989.
[13] S. L. Morrison et al., "Chimeric Human Antibody Molecules: Mouse Antigen-Binding Domains with Human Constant Region Domains," Proc. Natl. Acad. Sci. USA, **81**, pp. 6851-55 (1984).
[14] U.S. Patent No. 4,783,330, (Furie), "Monoclonal Antibodies to Activated Platelets," November 8, 1988.
[15] E. A. Lerner, "How to Make a Hybridoma," Yale J. Biol. Med., **54**, pp. 387-402 (1981).
[16] M. L. Gefter et al., "A Simple Method for Polyethylene Glycol-Promoted Hybridization of Mouse Myeloma Cells," Somatic Cell Genet., **3**, pp. 231-36 (1977).
[17] G. Galfre et al., "Antibodies to Major Histocompatibility Antigens Produced by Hybrid Cell Lines," Nature, **266**, pp. 550-52 (1977)
[18] L. Sauvage, Healing of Arterial Prostheses, Goal of Design and Clinical Use, (Cardiovascular Research Center, Seattle WA (1976)).
[19] T. Palabrica et al., "Thrombus Imaging in a Primate Model with Antibodies Specific for an External Membrane Protein of Activated Platelets," Proc. Natl. Acad. Sci. USA., **86**, pp. 1036-40 (1989).

## Claims

1. Use of an anti-PADGEM antibody, recombinant antibody, chimeric antibody or humanized antibody, or Fab fragment, Fab' fragment, F(ab')₂ fragment, F(v) fragment, heavy chain monomer, heavy chain dimer, heavy chain trimer, light chain monomer, light chain dimer, light chain trimer or dimer between one light chain and one heavy chain thereof that binds to PADGEM or a peptide that mimics the activity of anti-PADGEM antibodies in the manufacture of a medicament for inhibiting vascular narrowing at intravascular sites treated by angioplasty, endarterectomy or atherectomy, in natural and synthetic vascular grafts, at intravascular sites following deployment of vascular stents or cardiovascular devices, in arteriovenous fistulas, or at an intravascular site of platelet deposition.

2. A use as claimed in claim 1 wherein the vascular narrowing is associated with post-angioplasty reocclusion or restenosis, post-endarterectomy reocclusion or restenosis, post-atherectomy reocclusion or restenosis, post-vascular stent deployment reocclusion or restenosis, post-cardiovascular device insertion reocclusion or restenosis, or stenosis or occlusion within an arteriovenous fistula.

3. A use as claimed in claim 1 wherein the vascular narrowing is associated with a bypass graft prosthesis.

4. A use as claimed in any one of claims 1-3 wherein the anti-PADGEM antibody is a monoclonal antibody selected from the group consisting of monoclonal antibodies GA6 (ATCC No. HB10943), GE12 (ATCC No. HB10942) and AC11 (ATCC No. HB10941).

5. A use as claimed in any one of claims 1-4 wherein the anti-PADGEM antibody, recombinant antibody, chimeric antibody or humanized antibody, or Fab fragment, Fab' fragment, F(ab')₂ fragment, F(v) fragment, heavy chain monomer, heavy chain dimer, heavy chain trimer, light chain monomer, light chain dimer, light chain trimer or dimer between one light chain and one heavy chain thereof that binds to PADGEM or a peptide that mimics the activity of anti-PADGEM antibodies is for intravenous, intramuscular, subcutaneous or intra-arterial administration.

6. A use as claimed in any one of claims 1-5 wherein the anti-PADGEM antibody, recombinant antibody, chimeric antibody or humanized antibody, or Fab fragment, Fab' fragment, F(ab')₂ fragment, F(v) fragment, heavy chain monomer, heavy chain dimer, heavy chain trimer, light chain monomer, light chain dimer, light chain trimer or dimer between one light chain and one heavy chain thereof that binds to PADGEM or a peptide that mimics the activity of anti-PADGEM antibodies is for administration in a dosage of about 0.1 to about 5 mg/kg/day.

## Patentansprüche

1. Verwendung eines anti-PADGEM-Antikörpers, eines rekombinanten anti-PADGEM-Antikörpers, eines chimärischen anti-PADGEM-Antikörpers oder eines humanisierten anti-PADGEM-Antikörpers oder eines Fab-Fragments, Fab'-Fragments, F(ab')₂-Fragments, F(v)-Fragments, eines Schwerketten-Monomers, Schwerketten-Dimers, Schwerketten-Trimers, Leichtketten-Monomers, Leichtketten-Dimers, Leichtketten-Trimers oder eines Dimers zwischen einer leichten Kette und einer schweren Kette davon, das sich an PADGEM bindet oder eines Peptids, welches die Aktivität von anti-PADGEM-Antikörpern nachahmt, zur Herstellung eines Medikaments zur Hemmung von Gefäßverengung an intravaskulären Stellen, die mit Angioplastie, Endarteriektomie oder Atherektomie behandelt wurden, in natürlichen und synthetischen Gefäßtransplantaten, an intravaskulären Stellen nach dem Einsetzen von Gefäß-Stents oder kardiovaskulären Vorrichtungen, in arteriovenösen Fisteln oder an einer intravaskulären Stelle mit Thrombozytenablagerung.

2. Anwendung gemäß Anspruch 1, wobei die Gefäßverengung verbunden ist mit einer Reokklusion oder einer Restenose nach einer Angioplastie, mit einer Reokklusion oder einer Restenose nach einer Endarteriektomie, mit einer Reokklusion oder einer Restenose nach einer Atherektomie oder mit einer Reokklusion oder einer Restenose nach dem Einsetzen von Gefäß-Stents oder mit einer Reokklusion oder einer Restenose nach dem Einsetzen von kardiovaskulären Vorrichtungen, oder mit einer Stenose oder Okklusion in einer arteriovenösen Fistel.

3. Anwendung gemäß Anspruch 1, wobei die Gefäßverengung verbunden ist mit der Implantierung einer Bypass-Prothese.

4. Anwendung gemäß einem der Ansprüche 1-3, wobei der anti-PADGEM-Antikörper ein monoklonaler Antikörper ist, der ausgewählt ist aus der Gruppe bestehend aus den monoklonalen Antikörpern GA6 (ATCC Nr. HB10943), GE12 (ATCC Nr. HB10942) und AC11 (ATCC Nr. HB10941).

5. Anwendung gemäß einem der Ansprüche 1-4, wobei der anti-PADGEM-Antikörper, der rekombinante anti-PADGEM-Antikörper, der chimerische anti-PADGEM-Antikörper oder der humanisierte anti-PADGEM-Antikörper, oder das Fab-Fragment, das Fab'-Fragment, das F(ab')₂-Fragment, das F(v)-Fragment, das Schwerketten-Monomer, das Schwerketten-Dimer, das Schwerketten-Trimer, das Leichtketten-Monomer, das Leichtketten-Dimer, das Leichtketten-Trimer oder das Dimer zwischen einer leichten Kette und einer schweren Kette davon, das sich an PADGEM bindet oder ein Peptid, das die Aktivität von anti-PADGEM-Antikörpern nachahmt, zur intravenösen, intramuskulären, subkutanen oder intraarteriellen Verabreichung dienen.

6. Anwendung gemäß einem der Ansprüche 1-5, wobei der anti-PADGEM-Antikörper, der rekombinante anti-PADGEM-Antikörper, der chimerische anti-PADGEM-Antikörper oder der humanisierte anti-PADGEM-Antikörper, oder das Fab-Fragment, das Fab'-Fragment, das F(ab')₂-Fragment, das F(v)-Fragment, das Schwerketten-Monomer, das Schwerketten-Dimer, das Schwerketten-Trimer, das Leichtketten-Monomer, das Leichtketten-Dimer, das Leichtketten-Trimer oder das Dimer zwischen einer leichten Kette und einer schweren Kette davon, das sich an PADGEM bindet oder ein Peptid, das die Aktivität von anti-PADGEM-Antikörpern nachahmt, zur Verabreichung in einer Dosierung von ca. 0,1 bis ca. 5 mg/kg/Tag dienen.

## Revendications

1. Utilisation d'un anticorps, d'un anticorps recombinant, d'un anticorps chimère ou d'un anticorps humanisé, anti-PADGEM, ou d'un fragment Fab, fragment Fab', fragment F(ab')₂, fragment F(v), monomère de chaîne lourde, dimère de chaîne lourde, trimère de chaîne lourde, monomère de chaîne légère, dimère de chaîne légère, trimère de chaîne légère, ou dimère entre une chaîne légère et une chaîne lourde, de ceux-ci, qui se lie au PADGEM, ou d'un peptide qui simule l'activité d'anticorps anti-PADGEM, pour la fabrication d'un médicament permettant d'inhiber le rétrécissement vasculaire au niveau de sites intravasculaires traités par angioplastie, endartériectomie ou athérectomie, dans les greffons vasculaires naturels et synthétiques, au niveau de sites intravasculaires après déploiement d'endoprothèses vasculaires ou de dispositifs cardio-vasculaires, dans les fistules artério-veineuses, ou au niveau d'un site intravasculaire où s'est formé un dépôt de plaquettes.

2. Utilisation selon la revendication 1, où le rétrécissement vasculaire est associé à une réocclusion ou une resténose suivant une angioplastie, à une réocclusion ou resténose suivant une endardériectomie, à une réocclusion ou une resténose suivant une athérectomie, à une réocclusion ou resténose suivant le déploiement d'endoprothèses vasculaires, à une réocclusion ou resténose suivant l'insertion d'un dispositif cardio-vasculaire, ou à une occlusion ou une sténose à l'intérieur d'une fistule artério-veineuse.

3. Utilisation selon la revendication 1, où le rétrécissement vasculaire est associé à une prothèse d'un pontage par greffe.

4. Utilisation selon l'une quelconque des revendications 1 à 3, où l'anticorps anti-PADGEM est un anticorps monoclonal choisi dans le groupe constitué des anticorps monoclonaux GA6 (n° ATCC HB10943), GE 12 (n° ATCC HB10942) et AC11 (n° ATCC HB10941).

5. Utilisation selon l'une quelconque des revendications 1 à 4, où l'anticorps, l'anticorps recombinant, l'anticorps chimère ou l'anticorps humanisé, anti-PADGEM, ou le fragment Fab, fragment Fab', fragment F(ab')₂, fragment F(v), monomère de chaîne lourde, dimère de chaîne lourde, trimère de chaîne lourde, monomère de chaîne légère, dimère de chaîne légère, trimère de chaîne légère, ou dimère entre une chaîne légère et une chaîne lourde, de ceux-ci, qui se lie au PADGEM, ou un peptide qui simule l'activité d'anticorps anti-PADGEM, est destiné à une administration par voie intraveineuse, intramusculaire, sous-cutanée ou intra-artérielle.

6. Utilisation selon l'une quelconque des revendications 1 à 5, où l'anticorps, l'anticorps recombinant, l'anticorps chimère ou l'anticorps humanisé, anti-PADGEM, ou le fragment Fab, fragment Fab', fragment F(ab')₂, fragment F(v), monomère de chaîne lourde, dimère de chaîne lourde, trimère de chaîne lourde, monomère de chaîne légère, dimère de chaîne légère, trimère de chaîne légère, ou dimère entre une chaîne légère et une chaîne lourde, de ceux-ci, qui se lie au PADGEM, ou un peptide qui simule l'activité d'anticorps anti-PADGEM, est destiné à être administré avec une posologie d'environ 0,1 à environ 5 mg/kg/jour.
